# EUROPÄISCHE PATENTSCHRIFT

(11) **EP 3 449 260 B1**
(45) Veröffentlichungstag und Bekanntmachung des Hinweises auf die Patenterteilung: **30.12.2020**
(21) Anmeldenummer: 17722998.6
(22) Anmeldetag: 06.04.2017
(51) Int. Cl.: G01N 33/68, C07K 7/08

(54) **VERFAHREN ZUR IDENTIFIKATION VON INHIBITOREN DER PRIMÄREN NUKLEATION DER AMYLOID-BETA-AGGREGATION**
METHOD FOR IDENTIFYING INHIBITORS OF PRIMARY NUCLEATION OF AMYLOID-BETA AGGREGATION
PROCÉDÉ D'IDENTIFICATION D'INHIBITEURS DE LA NUCLÉATION PRIMAIRE DE L'AGRÉGATION DES BÊTA-AMYLOÏDES

(30) Priorität: 29.04.2016 DE 102016005169
(43) Veröffentlichungstag der Anmeldung: 06.03.2019
(73) Patentinhaber: Forschungszentrum Jülich GmbH, 52425 Jülich (DE)
(72) Erfinder: HOYER, Wolfgang, 40593 Düsseldorf (DE)
(86) Internationale Anmeldenummer: PCT/DE2017/000094
(87) Internationale Veröffentlichungsnummer: WO 2017/186203

(56) Entgegenhaltungen:
- US-A1- 2004 213 800
- US-A1- 2007 021 345
- US-B2- 8 323 647
- SEJIN LEE ET AL: "SYNTHESES OF BIOLOGICALLY ACTIVE AND COVALENTLY BONDED AMYLOID-B DIMERS (P1-118)", ALZHEIMER'S AND DEMENTIA, Bd. 10, Nr. 4, Suppl., 1. Juli 2014 (2014-07-01), Seite P344, XP055386251,
- LEE JOON SEOK ET AL: "High-Throughput Analysis of Alzheimer's .beta.-Amyloid Aggregation Using a Microfluidic Self-Assembly of Monomers", ANALYTICAL CHEMI, AMERICAN CHEMICAL SOCIETY, US, Bd. 81, Nr. 7, 1. Januar 2009 (2009-01-01) , Seiten 2751-2759, XP008145803, ISSN: 0003-2700, DOI: 10.1021/AC802701Z
- DOLPHIN GUNNAR T ET AL: "Designed amyloid beta peptide fibril - a tool for high-throughput screening of fibril inhibitors", CHEMMEDCHEM, WILEY - VCH VERLAG., WEINHEIM, DE, Bd. 2, Nr. 11, 1. November 2007 (2007-11-01), Seiten 1613-1623, XP002588461, ISSN: 1860-7179, DOI: 10.1002/CMDC.200700103
- HYE YUN KIM ET AL: "SYNTHETIC [beta]-AMYLOID DIMER IN ANTI-PARALLEL CONFORMATION INDUCES IN VIVO SYNAPTIC PLASTICITY DEFICITS", ALZHEIMER'S & DEMENTIA: THE JOURNAL OF THE ALZHEIMER'SASSOCIATION, Bd. 10, Nr. 4, 1. Juli 2014 (2014-07-01), Seite P870, XP055386252, US ISSN: 1552-5260, DOI: 10.1016/j.jalz.2014.05.1743
- FILIP HASECKE ET AL: "Origin of metastable oligomers and their effects on amyloid fibril self-assembly", CHEMICAL SCIENCE, vol. 9, no. 27, 1 January 2018 (2018-01-01), pages 5937-5948, XP055716391, United Kingdom ISSN: 2041-6520, DOI: 10.1039/C8SC01479E

### SEQUENCE LISTING

<110> Forschungszentrum Jülich GmbH
<120> verfahren zur Identifikation von Inhibitoren der primären Nukleation der Amyloid-Beta-Aggregation
<130> PT 1.2750
<160> 1
<170> PatentIn version 3.5
<210> 1
   <211> 20
   <212> PRT
   <213> peptidlinker
<400> 1

Gly Gly Gly Gly Ser Gly Gly Gly Gly Ser Gly Gly Gly Gly Ser Gly
1               5                   10                  15

Gly Gly Gly Ser
            20

## Beschreibung

Die Erfindung bezieht sich auf ein Verfahren zur Identifikation von Inhibitoren der primären Nukleation der Amyloid-Beta-Aggregation.

### Stand der Technik

Aufgrund der demographischen Entwicklung in den nächsten Jahrzehnten wird sich die Zahl der Personen, die an altersbedingten Erkrankungen leiden, erhöhen. Hier ist insbesondere die sogenannte Alzheimer-Krankheit (AD, Alzheimersche Demenz, lat. Morbus Alzheimer) zu nennen.

Bisher existiert kein Wirkstoff oder Medikament, das gegen die Ursachen von AD wirkt. Die bisher eingesetzten und zugelassenen Medikamente mildern einige der bei der Alzheimerschen Demenz auftretenden Symptome. Sie sind aber nicht in der Lage, den Krankheitsfortschritt zu verlangsamen oder eine Heilung herbeizuführen. Es existieren einige Substanzen, die im Tierversuch Erfolge bei der Prävention, aber nicht (unbedingt) bei der Behandlung von AD gezeigt haben.

Ein Merkmal der Alzheimer-Erkrankung sind extrazelluläre Ablagerungen des Amyloid-Beta-Peptids (Abeta-Peptid A-Beta-Peptid, Aß oder Aß-Peptid). Diese Ablagerung des A-Beta-Peptids in Plaques ist typischerweise in den Gehirnen von AD-Patienten *post mortem* festzustellen. Deshalb werden verschiedene Formen des A-Beta-Peptids - wie z.B. Fibrillen - für die Entstehung und das Fortschreiten der Krankheiten verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.

A-Beta-Monomere, als Bausteine der A-Beta-Oligomere, entstehen im menschlichen Körper ständig und sind vermutlich *per se* nicht toxisch. Es besteht sogar die Möglichkeit, dass Monomere eine positive Funktion besitzen. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomere könnten sich analog zu den Prionen vermehren und letztendlich zur Morbus Alzheimer-Krankheit führen.

Die aus dem Stand der Technik bekannten Substanzen verringern die Konzentration von A-Beta-Monomeren und/oder Oligomeren auf verschiedenste Art und Weise. So sind z.B. Gamma-Sekretase-Modulatoren bekannt, die im Tierversuch zur Prävention eingesetzt wurden.

Bei vielen Substanzen, die im Tierversuch positive Ergebnisse gezeigt haben, konnte diese Wirkung in klinischen Studien am Menschen nicht bestätigt werden. Wünschenswert wäre die Verhinderung der Bildung der ersten A-Beta Oligomere, also die Unterbindung der primären Nukleation. Dadurch könnten sich keine der toxischen Oligomere bilden.

Es besteht somit ein Bedarf an neuen Verbindungen wie Wirkstoffen und Inhibitoren, die sehr spezifisch und mit hoher Affinität an A-Beta-Oligomere entweder binden, und so deren Vermehrung verhindern oder direkt deren Bildung inhibieren.

Inhibitoren der Amyloid-Beta-Aggregation können im Prinzip erhalten werden, indem Moleküle mit einer Bindeaffinität für das Amyloid-Beta-Peptid selektiert werden. Beispielweise werden Antikörper mittels immunologischer Methoden und Peptide mittels Display-Selektiönsverfahren selektiert, welche anschließend hinsichtlich eines potentiellen inhibitorischen Effekts auf die Aggregation getestet werden.

Wünschenswert ist es, die Inhibitoren direkt über ihren Effekt auf die Aggregationsreaktion zu identifizieren. Dies ist mit Hilfe von kinetischen Aggregations-Assays möglich, wie es aus Arosio et al. bekannt ist (Arosio P., Vendruscolo, M., Dobson, C.M., Knowles, T.P.J. (2015). Chemical kinetics for drug discovery to combat protein aggregation diseases. Trends in Pharmacological Sciences. 35: 127-135).

Mit den aktuellen kinetischen Aggregations-Assays ist es nachteilig allerdings nur mit großem zeitlichen und materiellen Aufwand möglich, spezifisch Inhibitoren der primären Nukleation zu identifizieren, wie aus Habchi et al. bekannt ist (Habchi, J., Arosio, P., Perni, M., Costa, A.R., Yagi-Utsumi, M., Joshi, P., Chia, S., Cohen, S.I.A., Müller, M.B.D., Linse, S., Nollen, E.A.A., Dobson, C.M., Knowles, T.P.J. Vensdruscolo, V. (2016). An anticancer drug suppresses the primary nucleation reaction that initiates the production of the toxic Ab42 aggregates linked with Alzheimer's disease. Sci. Adv. 2, e1501244: 1-13).

Um einen inhibitorischen Effekt auf die primäre Nukleation für einen einzelnen Inhibitor-Kandidaten nachzuweisen, muss danach besonders nachteilig eine große Zahl von Aggregationsassays bei mehreren A-Beta- sowie Inhibitor-Konzentrationen durchgeführt werden, und das üblicherweise über den Zeitraum von mehreren Stunden, gefolgt von aufwändigen mathematischen Fit-Prozeduren.

Bei den nach diesem Stand der Technik erhaltenen Inhibitor-Kandidaten bleibt besonders nachteilig unklar, in welchen Reaktionsschritten der A-Beta-Aggregation die Inhibitoren überhaupt eingreifen.

So ist es aus dem Stand der Technik bekannt, dass häufig Moleküle selektiert werden, die zwar die Amyloidbildung hemmen, dafür aber zu einer vermehrten Bildung von potentiell toxischen A-Beta-Oligomeren führen. Moleküle, die spezifisch die primäre Nukleation inhibieren, können somit nur in aufwändigen, langwierigen Einzelfalluntersuchungen identifiziert werden.

### Aufgabe der Erfindung

Aufgabe der Erfindung ist es daher, ein einfacheres und schnelles, kostengünstiges Verfahren bereit zu stellen, mit dem Inhibitoren der primären Nukleation der Amyloid-Beta-Aggregation eindeutig zu identifizieren sind.

### Lösung der Aufgabe

Die Aufgabe wird gelöst mit dem Verfahren nach Patentanspruch 1 und gemäß Nebenanspruch. Vorteilhafte Ausgestaltungen hierzu ergeben sich aus den rückbezogenen Ansprüchen.

### Beschreibung der Erfindung

Während des Verfahrens zur Identifikation von Inhibitoren der primären Nukleation der Amyloid-Beta-Aggregation werden die nachfolgenden Schritte durchgeführt:
a) Eine A-Beta-Spezies wird in Lösung, insbesondere Puffer, vorgelegt;
b) Die Amyloid-Beta-Aggregation wird bestimmt.
   Das Verfahren ist dadurch gekennzeichnet, dass
c) als A-Beta-Spezies zwei Einheiten A-Beta-Monomer mit einem zwischen den A-Beta-Monomeren angeordneten Linker gewählt wird.

Mit primärer Nukleation wird die Entstehung jeglicher Oligomere bezeichnet.

Aus der US 2007/021345 A1 ist ein Verfahren zur Identifikation von Inhibitoren der primären Nukleation der Amyloid-Beta-Aggregation bekannt, mit den Schritten:
a) A-Beta-Spezies wird in Lösung bzw. Puffer vorgelegt;
b) Die Amyloid-Beta-Aggregation wird bestimmt; dadurch gekennzeichnet, dass
c) als A-Beta-Spezies A-Beta-Monomer gewählt wird.

Der Gegenstand der vorliegenden Erfindung unterscheidet sich von diesem bekannten Verfahren dadurch, dass als A-Beta-Spezies zwei Einheiten A-Beta-Monomer mit einem zwischen den A-Beta-Monomeren angeordneten Linker gewählt wird. Durch den Einsatz dieses Linkers kann ein schnelleres und kostengünstigeres Verfahren bereitgestellt werden. Die besonderen Vorteile des eingesetzten Linkers für das Verfahren werden im Folgenden beschrieben.

Die Amyloid-Beta-Aggregation und der Einfluss potentieller Inhibitoren kann auf verschiedene Weise über den Stand der Technik z. B. in Multiwellplatten nachgewiesen werden.

Es kann z. B. ein Fluoreszenz-Farbstoff zum Nachweis der Amyloidbildung der Lösung bzw. dem Puffer hinzugefügt werden. Der Anstieg des Fluoreszenzsignals als Nachweis der Amyloid-Beta-Aggregation wird dann bestimmt, beispielweise mittels des sogenannten Thioflavin T-Test.

Die folgenden weiteren Nachweisverfahren der Amyloid-Beta-Aggregation sind insbesondere möglich:
a. Fluoreszenzspektroskopie nach Zugabe von Farbstoff-Molekülen, deren Fluoreszenzeigenschaften (z. B. Fluoreszenzintensität, Fluoreszenzpolarisation) sich bei der Bindung an Amyloid-Beta-Aggregate ändern. Ein Beispiel ist der Farbstoff Thioflavin-T, der eine deutlich erhöhte Fluoreszenzintensität nach Bindung an Amyloid-Beta-Aggregate zeigt.
b. Fluoreszenzspektroskopie unter Verwendung von Abeta-Flexidimer-Molekülen, welche mit einem Farbstoff markiert sind, dessen Fluoreszenzeigenschaften (z. B. Fluoreszenzintensität, Fluoreszenzpolarisation) sich bei Bindung an Amyloid-Beta-Aggregate ändern.
c. Dynamische Lichtstreuung als etablierte Methode zum Nachweis von (Nano-)Partikeln und Proteinaggregaten.

Diese Messungen werden gegen die Zeit vorgenommen.

Im Falle b. können insbesondere Peptidlinker zwischen den A-Beta-Spezies gewählt werden, die mit Farbstoffen markiert sind.

Der Linker hat mehrere Vorteile. Er verkürzt die lag-Phase der Amyloid-Beta-Aggregation erheblich, vorzugsweise um mindestens 50 %, 60 %, 70 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % oder sogar 100 % im Vergleich zu der lag-Phase bei der Aggregation der entsprechend unverlinkten Monomere.

Vorteilhaft beginnt somit bei dem erfindungsgemäßen Verfahren die Amyloid-Beta-Aggregation sofort nach der Zugabe der verlinkten A-Beta-Monomere zum Puffer.

In Bezug auf das Verfahren bedeutet dies, dass wenn im Verfahren nach dem Stand der Technik mit unverlinkten Monomeren eine Zunahme des Fluoreszenzsignals nach etwa 10h Inkubationszeit erfolgte, dann wird mit dem Schritt c) des erfindungsgemäßen Verfahrens nach diesem Beispiel sichergestellt, dass damit die lag-Phase spätestens nach 2h bei einer um 80 % verkürzten lag-Phase bzw. spätestens nach einer 1h bei einer um 90 % verkürzten lag-Phase oder nach noch weniger Zeit beendet ist.

Es wurde erkannt, dass mit Schritt c) des erfindungsgemäßen Verfahrens vorteilhaft bewirkt wird, dass die lokale Konzentration an A-Beta-Spezies durch das Verlinken zweier A-Beta-Monomere erhöht und damit die Amyloid-Beta-Aggregation beschleunigt werden kann.

Es wurde erkannt, dass mit Schritt c) des erfindungsgemäßen Verfahrens, absolut betrachtet, auch vorteilhaft weniger Material an A-Beta-Spezies bei Verwendung verlinkter A-Beta-Monomere benötigt wird im Vergleich zu einem Assay, bei dem nicht verlinkte A-Beta-Monomere in Lösung verwendet werden. Das Verfahren ist somit kostengünstiger.

Hauptvorteil ist die deutlich verkürzte lag-Phase bei der, anders als im Stand der Technik, keine sekundären Prozesse mehr die primäre Nukleation überlagern. Daher kann mit dem erfindungsgemäßen Verfahren ein eindeutiger Nachweis der inhibitorischen Wirkung von Substanzen gegenüber der primären Nukleation bei der Amyloid-Beta-Aggregation erbracht werden.

Das erfindungsgemäße Verfahren nach den Schritten a) -c) ist dabei die Negativkontrolle eines Verfahrens, bei dem potentielle, auch aus dem Stand der Technik bereits bekannte, oder angenommene, Inhibitoren der primären Nukleation auf Wirksamkeit getestet werden können, um die Amyloid-Beta-Aggregation zu verhindern.

Als A-Beta-Spezies bzw. Monomere kommen insbesondere aber nicht ausschließlich A-Beta (1-42) sondern auch A-Beta (1-40) oder eine beliebige andere A-Beta-Spezies, wie z. B. Pyro-Glu-Abeta (3-40, 3-42) in Frage und können entsprechend des Assayansatzes ausgewählt werden. Das Verfahren ist anwendbar für alle bekannten A-Beta-Monomere.

Es ist denkbar, nicht nur die A-Beta-Monomere der Alzheimerschen Demenz und deren Aggregation auf diese Weise zu testen, sondern auch die Monomere anderer Proteinfehlfaltungserkrankungen. Dann werden die Monomere der bei diesen Erkrankungen auftretenden Proteine miteinander verlinkt.

Im Falle von AD können, müssen aber nicht, zwei identische Einheiten A-Beta-Spezies gewählt werden, welche mit dem Linker Kopf-zu-Schwanz oder anders verlinkt werden. Es können also auch A-Beta-Monomere verschiedener A-Beta-Spezies miteinander verlinkt werden, z. B. A-Beta (1-40) mit A-Beta (1-42).

Kopf-zu-Schwanz (engl. head-to-tail) bedeutet, dass der Linker zwischen dem C-Terminus einer ersten A-Beta-Monomereinheit und dem N-Terminus der zweiten A-Beta-Monomereinheit durch vorzugsweise eine kovalente Peptid-Bindung angeordnet ist.

Als Linker kann vorteilhaft ein Peptidlinker mit einer flexiblen Konformation (engl. intrinsically disordered conformation) gewählt werden. Um eine flexible Konformation zu ermöglichen und Wechselwirkungen des Linkers mit den A-Beta-Monomereinheiten zu unterbinden, kann der Linker vorteilhaft reich an polaren, ungeladenen Aminosäuren, wie z. B. Serin, Threonin und/oder Asparagin und/oder Glutamin und außerdem reich an kleinen Aminosäuren, wie z. B. Glycin und/oder Alanin und/oder Prolin sein. Insbesondere kann der Linker reich an Glycin und/oder Serin gewählt werden, und kann insbesondere auch ausschließlich aus Glycin und Serin bestehen. Eine solche Peptidsequenz ist beispielsweise bekannt aus Sejin Lee et al: "Syntheses of biologically active and covalently bonded amyloid-B dimers (P1-118)", Alzheimers's and Dementia, Bd. 10, Nr.4, Suppl, 1 Juli 2014, Seite P344 oder aus der US 8 323 647 B2.

Das Verfahren kann eine NMR-Spektroskopie als weiteren Schritt vorsehen, um zu zeigen, dass der Linker nicht in die Morphologie der Fibrillen im aggregierten Zustand eingreift und/oder die A-Beta-Monomere ihre native Konformation im Dimer erhalten.

Der Linker beeinflusst insbesondere nicht die intrinsische Unordnung der A-Beta-Einheiten (engl. intrinsically disordered conformation) in der Lösung bzw. im Puffer. Der Linker verändert insbesondere auch nicht die Morphologie der amyloiden Fibrillen im aggregierten Zustand.

Das Verfahren und die Kinetik der Aggregation von auf diese Weise verlinkten A-Beta-Einheiten ist im Wesentlichen durch die primären Nukleation geprägt und zwar insbesondere bei Konzentrationen an A-Beta-Spezies von etwa 1-50 microM im Puffer, vorzugsweise 1 microM, 2 microM, 5 microM, 10 microM, 20 microM bis etwa 50 microM, was zurückzuführen ist auf die Erhöhung der lokalen Konzentration an A-Beta. Dabei kann jeder Zwischenwert angenommen werden.

Mit dem Linker werden also die natürlichen Eigenschaften der Peptide nicht beeinflusst. Insbesondere werden die Eigenschaft der intrinsischen Ungeordnetheit vor der Ausbildung der Konformation der Fibrillen und/oder Aggregate durch den linker nicht beeinflusst.

Das Verfahren kann einen Schritt aufweisen, bei dem die Konformation der verlinkten A-Beta-Spezies mit der der unverlinkten Einheiten verglichen wird, beispielweise durch NMR-Messungen.

Die verlinkten Einheiten dienen vorteilhaft als Modellsubstanz für die unverlinkten Einheiten.

Das Verfahren kann einen Schritt aufweisen, bei dem untersucht wird, dass bzw. ob die verlinkten A-Beta-Spezies dieselbe Form Fibrillen ausbilden, wie die unverlinkten A-Beta-Spezies. Hierzu kann z. B. Rasterkraftmikroskopie durchgeführt werden.

Das erfindungsgemäße Verfahren ist geeignet für ein Hochdurchsatz-Screening, z. B. in Multiwell-Mikrotiterplatten.

Das Verfahren wird insbesondere zur Überprüfung der Kinetik der primären Nukleation durchgeführt. Hierzu kann ein Mikrotiter basierter Assay mit und ohne Inhibitoren der Amyloid-Beta-Aggregation durchgeführt werden.

Als Linker kann insbesondere ein Peptidlinker mit der Sequenz (Glyₓ-Ser_{y})ₙ gewählt werden mit x=1-4, y=1 und n=1-10.

An Stelle des Serin kann Threonin und/oder Asparagin und/oder Glutamin gewählt werden. Diese Aminosäuren erhalten vorteilhaft die Stabilität.

An Stelle des Glycin kann Alanin und/oder Prolin gewählt werden.

Als Faustregel sollten im linker etwa 10-90 % der Anzahl an Aminosäuren wie in der verlinkten A-Beta-Spezies vorliegen. Generell sollte der linker im Falle von Proteinfehlfaltungserkrankungen etwa 10-90 % der Aminosäureanzahl der die Aggregation auslösenden Monomereinheiten umfassen. Im Falle von A-Beta (1-40) umfasst der Linker dann etwa 4-36 Aminosäuren. Insbesondere umfasst der Linker 20-80 %, 30-70 % oder 40-60 % der Aminosäureanzahl der die Aggregation auslösenden Monomereinheiten.

Als weitere Faustregel sollten im Linker insbesondere etwa 50 % ± 5 % der Anzahl an Aminosäuren wie in der verlinkten A-Beta-Spezies vorliegen. Generell sollte der linker im Falle von Proteinfehlfaltungserkrankungen etwa 50 % ± 5 % der Aminosäureanzahl der die Aggregation auslösenden Monomereinheiten aufweisen.

Insbesondere hat sich als Linker ein Peptidlinker mit der Sequenz (Gly₄-Ser)₄ bewährt, wobei die oben genannten Bedingungen des Austauschs gegen anderen Aminosäuren gelten können.

Diese Linker verkürzen vorteilhaft die lag-Phase der Amyloid-Beta-Aggregation der Alzheimerschen Demenz besonders stark, insbesondere, aber nicht ausschließlich, bei A-Beta (1-40) und/oder A-Beta (1-42) als zu verlinkende A-Beta-Spezies.

Die Konzentration, in der die verlinkte A-Beta-Spezies vorgelegt werden soll, sollte insbesondere etwa >2 mikroM betragen. Dann sind die Verkürzung der lag-Phase und die Dominanz der primären Nukleation besonders ausgeprägt.

Mit dem vorgenannten Verfahren und der damit einhergehenden Verkürzung der lag-Phase wird sichergestellt, dass die Amyloid-Beta-Aggregation sofort beginnt und nachweisbar ist. Dadurch ist der Einfluss potentieller Wirkstoffkandidaten auf die Kinetik der primären Nukleation nachweisbar.

Vorgenannte erfindungsgemäße linker zwischen den A-Beta-Spezies oder Monomereinheiten anderer Proteinfehlfaltungserkrankungen verringern die lag-Phase einer (Amyloid-Beta-)Aggregation deutlich, vorzugsweise um mindestens 80 %.

Eine zu testende Substanz, z. B. ein Inhibitor wird hinzugefügt und positiv gewertet, wenn er entweder die Höhe des Fluoreszenzsignals begrenzt und/oder den zeitlichen Anstieg des Fluoreszenzsignals verlangsamt gegenüber des Signals der Negativkontrolle.

Der flexible linker mit der Aminosäuresequenz ggggsggggsggggsggggs als Peptid gemäß der SEQ ID NO: 1 ist beispielhaft und besonders relevant, da er vorteilhaft die erforderlichen Eigenschaften aufweist. Dieser linker findet Verwendung bei der Identifikation von Wirkstoffen und Inhibitoren der primären Nukleation der Amyloid-Beta-Aggregation, insbesondere aus A-Beta (1-40) und/oder A-Beta (1-42) und/oder anderer Monomere von Proteinfehlfaltungserkrankungen, wobei wiederum die oben genannten Regeln der Austauschbarkeit gegen andere Aminosäuren gelten sollen.

Der Linker beeinflusst vorteilhaft nicht die Faltblattstruktur der Amyloid-Beta-Aggregation.

Dann ist über den Stand der Technik, hier einem Thioflavin-Test die Amyloid-Beta-Aggregation in Multiwellplatten über Messung des Anstiegs der Fluoreszenzsignale über die Zeit und der Einfluss potentieller Inhibitoren nachweisbar.

Die Substanzen, insbesondere die Wirkstoffe bzw. Inhibitoren, welche die A-Beta-Aggregation hemmen, sind entsprechend vielversprechende Wirkstoffkandidaten für die Therapie der Alzheimer-Krankheit, da mit dem erfindungsgemäßen Verfahren die besonders toxischen Oligomere erst gar nicht entstehen. Das Verfahren führt in diesem Sinne dazu die Sekundärprozesse der Amyloid-Beta-Aggregation zeitlich zu überspringen.

Besonders die Inhibitoren der allerersten Schritte der Aggregationsreaktion, der sogenannten primären Nukleation sind dabei auch als Wirkstoffe interessant. Solche Inhibitoren unterbinden potentiell die Bildung der besonders toxischen A-Beta-Oligomere der Alzheimerschen Demenz.

Mit dem erfindungsgemäßen Verfahren gelingt es auf besonders einfache und beschleunigte Weise, zuverlässige Inhibitoren der primären Nukleation der Amyloid-Beta-Aggregation zu identifizieren durch entsprechende Verkürzung der lag-Phase und somit Wirkstoffe gegen die Bildung der toxischen Oligomere kostengünstig aufzufinden.

Dabei können selbstverständlich auch die bereits im Stand der Technik identifizierten Moleküle wie Peptide, Antikörper, Wirkstoffe zur Behandlung von Krebs, wie beispielweise Bexaroten, in dem verkürzten erfindungsgemäßen Assay angewendet werden und auf ihre Wirksamkeit bei der Unterbindung der Bildung toxischer Oligomere untersucht werden. Diese können als Positivkontrolle eingesetzt werden.

Hierzu wurde im Rahmen der Erfindung erkannt, dass die Amyloid-Beta-Aggregation eine komplexe, aus mehreren Schritten bestehende Reaktion ist, in der nachgelagerte, sekundäre Prozesse den beobachtbaren Reaktionsverlauf gängiger Assay nach dem Stand der Technik dominieren. Es wurde außerdem erkannt, dass diese nachgelagerten Prozesse während der lag-Phase auftreten.

Entsprechend wurde erfindungsgemäß ein kinetischer Aggregations-Assay entwickelt, der nicht durch sekundäre Prozesse sondern durch die primäre Nukleation dominiert wird, durch Verkürzung der lag-Phase.

Anstelle von monomerem A-Beta nutzt der Assay, bzw. die erfindungsgemäßen Verfahren ein neu entwickeltes dimeres Konstrukt mit dem Arbeitstitel "Abeta-FlexiDimer", in welchem zwei A-Beta-Monomere über einen flexiblen Peptidlinker miteinander verbunden sind. Hierzu können beispielweise zwei Einheiten der häufigsten A-Beta-Variante, das A-Beta (1-40), eingesetzt werden. Dimere anderer A-Beta-Varianten, wie z. B. A-Beta (1-42) oder modifizierter Versionen, auch kürzerer Versionen, und Mischungen hieraus, sind von Interesse und daher Gegenstand der Erfindung. Lediglich beispielhaft kann ein Monomer A-Beta (1-40) mit einem Monomer A-Beta (1-42) verlinkt werden.

Die relativ große Länge des Linkers wurde gewählt, damit die A-Beta-Einheiten im dimeren Konstrukt nicht durch Interaktionen mit dem fusionierten Nachbarn in der Ausbildung der gewöhnlichen Amyloidstruktur gehindert werden, was durch Rasterkraftmikroskopie-Messungen bestätigt werden kann.

Verglichen mit A-Beta-Monomer zeigt Abeta-FlexiDimer vorteilhaft ein verbessertes Verhalten in kinetischen Aggregations-Assays, hier dem erfindungsgemäßen Verfahren. Es ist vorteilhaft gekennzeichnet dadurch, dass es die lag-Phase fast vollständig eliminiert. Hierzu kann die Konzentration ab 1 mikroM bis etwa 50 mikroM, vorzugsweise etwa 5 mikroM im Puffer betragen. Dadurch wird vorteilhaft bewirkt, dass es zu der deutlichen Zeitersparnis bei der Durchführung des Test-Verfahrens kommt. Außerdem lässt sich auf diese Weise ein zugegebener Wirkstoff als ein Wirkstoff identifizieren, der tatsächlich die primäre Nukleation inhibiert und nicht erst zu einem späteren Zeitpunkt in die weitere Aggregation eingreift, bei gleichzeitig verringerter A-Beta-Monomer-Konzentrationen.

Diese positiven Effekte sind aus dem Stand der Technik zu Linkern, wie z. B. aus Chen et al. (Chen, X., Zaro, J., Shen, W.-C. (2013). Fusion protein linkers: Property, Design and Functionality. Adv Drug Deliv Rev 65(10): 1357-1369) oder aus Chichilli et al. (Chichilli, V.P.R., Kumar, V., Sivaram J. (2013). Protein Science 22:153-167) nicht zu erwarten gewesen.

Auf diese Weise werden die Inhibitoren identifiziert, die die Bildung toxischer Oligomere verhindern und als Wirkstoffe gegen die entsprechenden Proteinfehlfaltungserkrankungen in Frage kommen.

Die Erfindung ist hierauf nicht beschränkt. Vielmehr betrifft die Erfindung weitere Verfahren, die sich auf die Aggregation von Monomeren von Proteinfehlfaltungserkrankung im Generellen beziehen, und zwar insbesondere:
Verfahren zur Untersuchung der Aggregation von Amyloid-Beta-Monomeren von Proteinfehlfaltungserkrankungen, mit den Schritten:
a) Eine Amyloid-Beta-Spezies wird in Lösung bzw. Puffer vorgelegt;
b) Die Aggregation der Amyloid-Beta-Monomere wird bestimmt;
dadurch gekennzeichnet, dass
als A-Beta-Spezies zwei Einheiten A-Beta-Monomer mit einem zwischen den A-Beta-Monomeren angeordneten Linker gewählt wird.

Der Linker weist die Merkmale wie beschrieben auf.

Es versteht sich, dass hierzu auch A-Beta-Monomere, wie das genannte A-Beta (1-42) und/oder A-Beta (1-40) und/oder andere bei der Alzheimerschen Demenz auftretende Monomere auf ihr Aggregationsverhalten hin getestet werden können. Außerdem kann jedes andere Monomer einer anderen Proteinfehlfaltungserkrankung auf die erfindungsgemäß Weise gemäß der offenbarten Verfahren getestet werden, sowie ein KIT bzw. ein Peptidlinker hierzu beansprucht werden.

### Ausführungsbeispiele

Im Weiteren wird die Erfindung an Hand von Ausführungsbeispielen und der beigefügten Figuren näher erläutert, ohne dass es hierdurch zu einer Beschränkung der Erfindung auf Amyloid-Beta-Aggregation kommt.

Es zeigen:
- Figur 1:: Kinetik des erfindungsgemäßen Verfahrens mit erfindungsgemäßen Dimer. A Aggregation von Abeta-Flexi-Dimer bei unterschiedlichen Konzentrationen. B Übergang zwischen der primären Nukleation und den Sekundärprozessen in Abhängigkeit von der Konzentration.
- Figur 2:: Modellvergleich zwischen A-Beta (1-40) Amyloid Formation mit der Fibrillenformation aus dem FlexiDimer.

In eine Mikrotiterplatte wird eine 25 mikroM Lösung von Abeta-FlexiDimer in einem Puffer, z. B. in 20 mikroM Natriumphosphat, pH 7,4, 50 mikroM NaCl, vorgelegt. Ein Farbstoff für den Nachweis der Amyloidbildung, z. B. Thioflavin T, wird hinzugegeben.

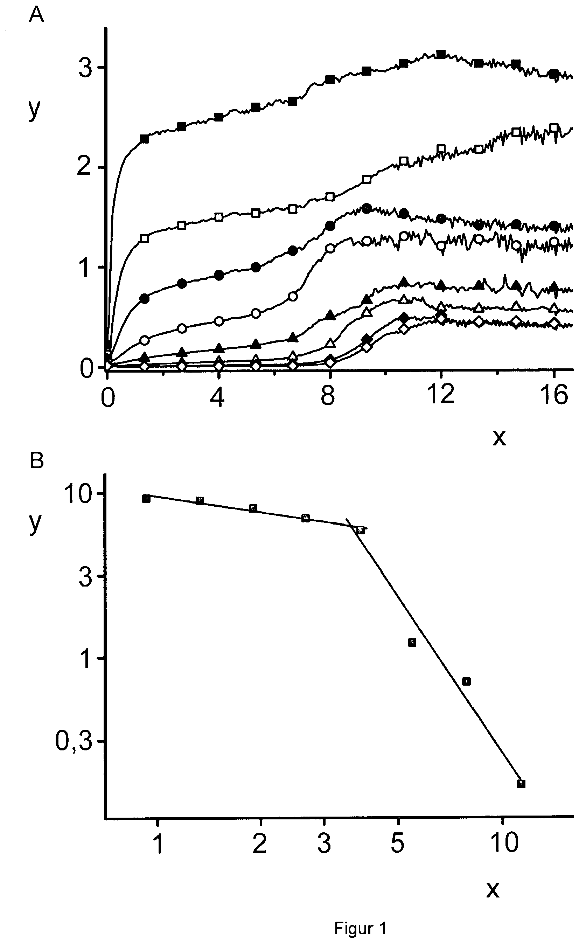

Der Einfluss von A-Beta-Monomer-FlexiDimer auf die Verkürzung der lag-Phase ist in der Figur 1 gezeigt.

A Auf der x-Achse ist die Zeit in Stunden und auf der y-Achse die Fluoreszenzintensität als Maß für die Aggregation aufgetragen. Die Aggregation von Abeta-FlexiDimer ist bei Anfangskonzentrationen >1 mikroM direkt ab dem Beginn des Aggregations-Assays ohne Verzögerungszeit nachweisbar (Figur 1A, offene Raute 0,9 mikroM, geschlossene Raute 1,3 mikroM, offenes Dreieck 1,9 mikroM, geschlossenes Dreieck 2,7 mikroM, offene Kreise 3,9 mikroM, geschlossene Kreise 5,5 mikroM, offene Quadrate 8 mikroM, geschlossene Quadrate 11 mikroM).

Dieses Verhalten ist in Übereinstimmung mit einem Übergang von dominanten sekundären Prozessen zu dominanter primärer Nukleation bei AbetaFlexidimer-Konzentrationen >2 mikroM (Figur 1B). In der Figur 1B ist ein doppellogarithmischer Plot von t1/2 (in Stunden) gegen die Konzentration an FlexiDimer (in mikroM) aufgetragen. Ab einer Konzentration von etwa 3 mikroM ist die primäre Nukleation der dominante Prozess bei der Amyloid-Beta-Aggregation von Abeta-FlexiDimer. Eine Steigung von etwa -0,3 zeigt die Dominanz von Sekundärprozessen und eine Steigung von etwa -3 zeigt die Dominanz der primären Nukleation an.

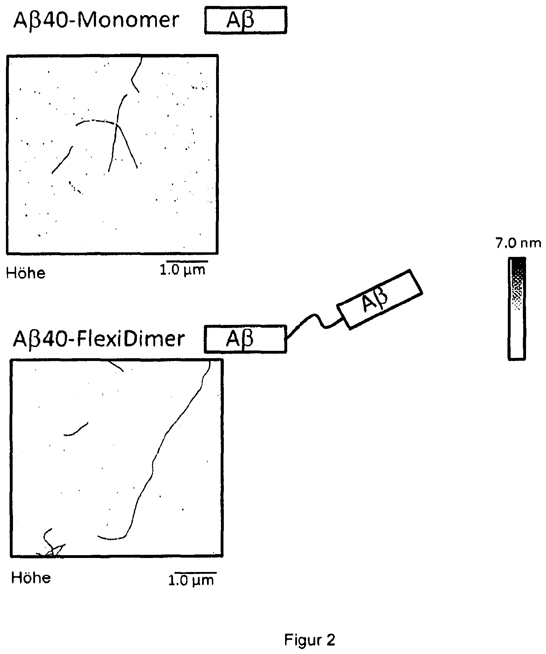

Figur 2 zeigt mittels Rasterkraftmikroskopie, dass das FlexiDimer Fibrillen derselben Morphologie bildet, wie das A-Beta-Monomer, hier für den Fall von A-Beta (1-40).

Inhibitor-Kandidaten werden zu den einzelnen Wells zugegeben. Die Mikrotiterplatte wird bei 37 °C in einem Fluoreszenz-Leser inkubiert und in einem Zeitraum von wenigen Minuten wird in regelmäßigen Intervallen die Fluoreszenz bestimmt.

Inhibitoren werden dadurch identifiziert, dass sie den zeitlichen Anstieg des Fluoreszenzsignals aufhalten oder verlangsamen (nicht dargestellt).

### Weitere Ausführungsbeispiele

1. In eine Mikrotiterplatte wird eine 25 mikroM Lösung von Abeta-FlexiDimer in einem Puffer, z. B. in 20 mikroM Natriumphosphat, pH 7,4, 50 mikroM NaCl, vorgelegt. Ein Farbstoff für den Nachweis der Amyloidbildung, z. B. Thioflavin T, wird hinzugegeben. Die Fluoreszenzintensität in Abwesenheit und Gegenwart von Inhibitoren wird mit einem Fluoreszenz-Leser gemessen.
2. Abeta-Flexidimer wird mit einem geeigneten Farbstoffmolekül markiert, welches durch eine Erhöhung der Fluoreszenzpolarisation die Amyloid-Beta-Aggregation anzeigt. In eine Mikrotiterplatte wird eine 25 mikroM Lösung des markierten Abeta-FlexiDimer in einem Puffer, z. B. in 20 mikroM Natriumphosphat, pH 7,4, 50 mikroM NaCl, vorgelegt. Die Fluoreszenzpolarisation in Abwesenheit und Gegenwart von Inhibitoren wird mit einem Fluoreszenz-Leser gemessen.
3. In eine Mikrotiterplatte wird eine 25 mikroM Lösung von Abeta-FlexiDimer in einem Puffer, z. B. in 20 mikroM Natriumphosphat, pH 7,4, 50 mikroM NaCl, vorgelegt. Die Lichtstreuung in Abwesenheit und Gegenwart von Inhibitoren wird mit einem Lichtstreudetektor gemessen.

## Patentansprüche

1. Verfahren zur Identifikation von Inhibitoren der primären Nukleation der Amyloid-Beta-Aggregation, mit den Schritten:
a) A-Beta-Spezies wird in Lösung bzw. Puffer vorgelegt;
b) die Amyloid-Beta-Aggregation wird bestimmt; **dadurch gekennzeichnet, dass**
c) als A-Beta-Spezies zwei Einheiten A-Beta-Monomer (1-40) und/oder A-Beta-Monomer (1-42) mit einem zwischen den A-Beta-Monomeren angeordneten Linker gewählt wird, wobei als Linker ein Peptidlinker mit der Sequenz (Glyₓ-Ser_{y})ₙ gewählt wird, mit x = 1-4, y = 1 und n = 1-10 und wobei der Linker 10 bis 90% der Anzahl an Aminosäuren wie die verlinkten A-Beta-Spezies aufweist und
wobei der Linker zwischen dem C-Terminus des ersten A-Beta- Monomers und dem N-Terminus des zweiten A-Beta-Monomers angeordnet ist, und wobei als Linker ein Peptidlinker mit flexibler Konformation gewählt wird, und wobei als Linker, der sich zwischen den zwei Einheiten der A-Beta-Monomere befindet, ein Peptid gewählt wird, welches die lag-Phase der Amyloid-Beta-Aggregation um mindestens 50 % im Vergleich zu der lag-Phase bei der Aggregation der entsprechend unverlinkten Monomere verringert,
d) ein Fluoreszenz-Farbstoff zum Nachweis der Amyloidbildung zur Lösung bzw. Puffer hinzugefügt wird und der Anstieg des Fluoreszenzsignals als Nachweis der Amyloid-Beta-Aggregation gemessen wird
e) ein zu testender Inhibitor hinzugefügt wird und positiv gewertet wird, wenn er die Höhe des Fluoreszenzsignals begrenzt oder den zeitlichen Anstieg des Fluoreszenzsignals verlangsamt.

2. Verfahren nach vorherigem Anspruch,
**dadurch gekennzeichnet, dass**
in Schritt d) kein Fluoreszenzfarbstoff zugefügt wird und die Amyloid-Beta-Aggregation durch Lichtstreuung nachgewiesen wird.

3. Verfahren nach einem der vorherigen Ansprüche,
**dadurch gekennzeichnet, dass**
zwei identische oder zwei nicht identische Einheiten A-Beta-Monomer gewählt werden.

4. Verfahren nach einem der vorherigen Ansprüche,
**dadurch gekennzeichnet, dass**
als Linker, der sich zwischen den zwei Einheiten der A-Beta-Monomere befindet, ein Peptid gewählt wird, welches die lag-Phase der Amyloid-Beta-Aggregation um 80 %, insbesondere 90 % oder sogar 100 % verringert.

5. Verfahren nach einem der vorherigen Ansprüche,
**dadurch gekennzeichnet, dass**
als Linker ein Peptidlinker mit der Sequenz (Gly₄-Ser)₄ gewählt wird.

6. Verfahren zur Untersuchung der Aggregation von Amyloid-Beta-Monomeren, mit den Schritten:
- eine Amyloid-Beta-Spezies wird in Lösung bzw. Puffer vorgelegt;
- die Aggregation der Amyloid-Beta-Monomere wird bestimmt;
- **dadurch gekennzeichnet, dass**
als A-Beta-Spezies zwei Einheiten A-Beta-Monomer (1-40) und/oder A-Beta-Monomer (1-42) mit einem zwischen den A-Beta-Monomeren angeordneten Linker gewählt wird, wobei als Linker ein Peptidlinker mit flexibler Konformation mit der Sequenz (Glyₓ-Ser_{y})ₙ gewählt wird, mit x = 1-4, y = 1 und n = 1-10.

## Claims

1. A method for identifying inhibitors of the primary nucleation of the amyloid-beta aggregation, with the steps:
a) A-beta species is provided in solution and buffer, respectively;
b) the amyloid-beta aggregation is determined;
**characterized in that**
c) two units A-beta monomer (1-40) and/or A-beta monomer (1-42) having a linker arranged between the A-beta monomers are selected as A-beta species, wherein a peptide linker with the sequence (Glyₓ-Ser_{y})ₙ is selected as linker, with x = 1-4, y = 1 and n = 1-10, and
wherein the linker has 10 to 90% of the number of amino acids such as the linked A-beta species, and wherein the linker is arranged between the C-terminus of the first A-beta monomer and the N-terminus of the second A-beta monomer, and wherein a peptide linker with flexible conformation is selected as linker, and wherein a peptide is selected as linker located between the two units of the A-beta monomers, which peptide reduces the lag phase of the amyloid-beta aggregation by at least 50% compared to the lag phase when aggregating the corresponding unlinked monomers,
d) a fluorescent dye for the detection of the amyloid formation is added to the solution and buffer, respectively, and the increase in the fluorescence signal is measured as evidence of the amyloid-beta aggregation,
e) an inhibitor to be tested is added and is evaluated as positive if it limits the level of the fluorescence signal or slows down the temporal rise in the fluorescence signal.

2. A method according to the preceding claim,
**characterized in that**
in step d), no fluorescent dye is added and the amyloid-beta aggregation is detected by light scattering.

3. A method according to any one of the preceding claims,
**characterized in that**
two identical or two non-identical units A-beta monomer are selected.

4. A method according to any one of the preceding claims,
**characterized in that**
a peptide is selected as linker located between the two units of the A-beta monomers, which peptide reduces the lag phase of the amyloid-beta aggregation by 80%, in particular 90% or even 100%.

5. A method according to any one of the preceding claims,
**characterized in that**
a peptide linker with the sequence (Gly₄-Ser)₄ is selected as linker.

6. A method for investigating the aggregation of amyloid-beta monomers, with the steps:
- an amyloid-beta species is provided in solution and buffer, respectively;
- the aggregation of the amyloid-beta monomers is determined;
**characterized in that**
two units A-beta monomer (1-40) and/or A-beta monomer (1-42) with a linker, which is arranged between the A-beta monomers, are selected as A-beta species, wherein a peptide linker with flexible conformation with the sequence (Glyₓ-Ser_{y})ₙ is selected as linker, with x = 1-4, y = 1 and n = 1-10.

## Revendications

1. Procédé d'identification d'inhibiteurs de la nucléation primaire de l'agrégation des bêta-amyloïdes, comprenant les stades :
a) on met au début une espèce de bêta-A en solution ou dans un tampon ;
b) on détermine l'agrégation des bêta-amyloïdes ; **caractérisé en ce que**
c) comme espèce de bêta-A, on choisit deux motifs de monomère de bêta-A (1-40) et/ou de monomère de bêta-A (1-42) ayant un linker disposé entre les monomères de bêta-A, un linker peptidique ayant la séquence (Glyₓ-Ser_{y})ₙ étant choisi comme linker, avec x = 1 à 4, y = 1 et n = 1 à 10 et le linker ayant de 10 à 90% du nombre des acides aminés comme l'espèce de bêta-A linkérisée et
dans lequel le linker est disposé entre la terminaison C du premier monomère de bêta-A et la terminaison N du deuxième monomère de bêta-A,
et dans lequel on choisit, comme linker, un linker peptique de conformation souple,
et dans lequel on choisit, comme linker, qui se trouve entre les deux motifs des monomères de bêta-A, un peptide, qui diminue la phase de latence de l'agrégation des bêta-amyloïdes d'au moins 50% par rapport à la phase de latence lors de l'agrégation des monomères non linkérisés correspondants,
d) on ajoute à la solution ou au tampon un colorant de fluorescence pour déceler la formation d'amyloïdes et on mesure l'augmentation du signal de fluorescence comme preuve de l'agrégation des bêta-amyloïdes,
e) on ajoute un inhibiteur à tester et on l'évalue positivement, s'il limite le niveau du signal de fluorescence ou ralentit l'augmentation en fonction du temps du signal de fluorescence.

2. Procédé suivant la revendication précédente,
**caractérisé en ce qu'**
au stade d), on n'ajoute pas de colorant de fluorescence et on décèle l'agrégation de bêta-amyloïdes par dispersion de la lumière.

3. Procédé suivant l'une des revendications précédentes,
**caractérisé en ce que**
l'on choisit deux motifs identiques ou deux motifs non identiques de monomères de bêta-A.

4. Procédé suivant l'une des revendications précédentes,
**caractérisé en ce que**
l'on choisit comme linker, qui se trouve entre les deux motifs des monomères de bêta-A, un peptide, qui diminue la phase de latence de l'agrégation des bêta-amyloïdes de 80%, notamment de 90% ou même de 100%.

5. Procédé suivant l'une des revendications précédentes,
**caractérisé en ce que**
l'on choisit comme linker un linker peptidique ayant la séquence (Gly₄-Ser)₄.

6. Procédé d'étude de l'agrégation de monomères de bêta-amyloïde, comprenant les stades :
- on met au début une espèce de bêta-A en solution ou dans un tampon ;
- on détermine l'agrégation des monomères de bêta-amyloïde ;
- **caractérisé en ce que**
comme espèce de bêta-A, on choisit deux motifs de monomère de bêta-A (1-40) et/ou de monomère de bêta-A (1-42) ayant un linker disposé entre les monomères de bêta-A, un linker peptidique de conformation souple ayant la séquence (Glyₓ-Ser_{y})ₙ étant choisi comme linker, avec x = 1 à 4, y = 1 et n = 1 à 10.
